(19) Europäisches Patentamt
European Patent Office
Office européen des brevets

(11) Veröffentlichungsnummer: **0 292 819 B1**

(12) **EUROPÄISCHE PATENTSCHRIFT**

(45) Veröffentlichungstag der Patentschrift: **08.04.92**

(51) Int. Cl.$^5$: **C07D 209/60**, A61K 31/40

(21) Anmeldenummer: **88107782.0**

(22) Anmeldetag: **14.05.88**

# (54) 5-Phenyl-1,2,3a,4,5,9b-hexahydro-3H-benz [e] indole, ihre Herstellung und Berwendung als Arzneimittel.

(30) Priorität: **23.05.87 DE 3717395**

(43) Veröffentlichungstag der Anmeldung:
**30.11.88 Patentblatt 88/48**

(45) Bekanntmachung des Hinweises auf die Patenterteilung:
**08.04.92 Patentblatt 92/15**

(84) Benannte Vertragsstaaten:
**AT BE CH DE ES FR GB IT LI NL**

(56) Entgegenhaltungen:
**EP-A- 0 130 069**
**EP-A- 0 201 085**
**EP-A- 0 219 055**
**DE-A- 2 235 667**
**DE-A- 3 632 589**

(73) Patentinhaber: **BASF Aktiengesellschaft**
**Carl-Bosch-Strasse 38**
**W-6700 Ludwigshafen(DE)**

(72) Erfinder: **Steiner, Gerd, Dr.**
**Oberer Waldweg 1**
**W-6719 Kirchheim(DE)**
Erfinder: **Himmele, Walter, Dr.**
**Eichenweg 14**
**W-6909 Walldorf(DE)**
Erfinder: **Buschmann, Ernst, Dr.**
**Georg-Ludwig-Krebs-strasse 10**
**W-6700 Ludwigshafen(DE)**
Erfinder: **Teschendorf, Hans-Juergen, Dr.**
**Georg-Nuss-Strasse 5**
**W-6724 Dudenhofen(DE)**
Erfinder: **Weifenbach, Harald, Dr.**
**Londoner Ring 71**
**W-6700 Ludwigshafen(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Die Erfindung betrifft neue 5-Phenyl-1,2,3a,4,5,9b-hexahydro-3H-benz[e]indole, Verfahren zu deren Herstellung sowie diese enthaltende therapeutische Mittel und deren Verwendung bei der Bekämpfung von Krankheiten.

Es sind bereits tricyclische Pyrrolo[2,1-a]isochinolin-Derivate bzw. Phenyl-benz[h]isochinolin-Derivate beschrieben worden, die antidepressive Wirkung besitzen sollen [EP-OS 130 069, EP-OS 201 085].

Es wurde nun gefunden, daß 5-Phenyl-1,2,3a,4,5,9b-hexahydro-3H-benz[e]indole der Formel I

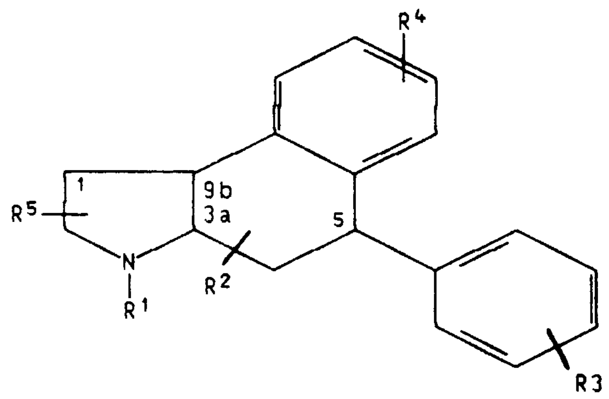

in der

| | |
|---|---|
| $R^1$ | Wasserstoff oder $C_{1-6}$-Alkyl |
| $R^2$ | Wasserstoff oder $C_{1-3}$-Alkyl in der 3a- oder 4-Stellung |
| $R^3$ und $R^4$ | Wasserstoff, Hydroxy, Halogen, $C_{1-3}$-Alkyl, $C_{1-3}$-Alkoxy, $C_{1-3}$-Alkylthio oder Trifluormethyl, Acetamino, Amino und |
| $R^5$ | Wasserstoff oder $C_{1-3}$-Alkyl |

bedeuten und ihre Salze mit physiologisch verträglichen Säuren wertvolle pharmakologische Eigenschaften besitzen.

Als besonders günstig haben sich die Verbindungen erwiesen, in denen $R^1$ Wasserstoff, oder vorzugsweise Methyl, Ethyl oder n-Propyl ist, $R^2$, $R^4$ und $R^5$ Wasserstoff bedeuten und $R^3$ Fluor, Chlor, Trifluormethyl, Methoxy, Thiomethyl, Methyl, Hydroxy, Acetamino, Amino oder insbesondere Wasserstoff ist.

Es sei darauf hingewiesen, daß die erfindungsgemäßen Verbindungen mindestens 3 chirale Zentren in den Positionen 3a, 5 und 9a besitzen und deshalb als unterschiedliche Diastereomere auftreten, die z.B. durch fraktionierte Kristallisation getrennt werden können. Dabei erwiesen sich die Verbindungen Ia mit cisständiger Pyrrolidin-Anellierung als besonders wirksam:

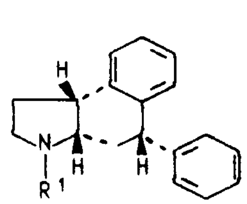

Ia

Insbesondere sind folgende Verbindungen zu nennen:
(±)-3aS(R),5S(R),9bR(S)-3-Methyl-5-phenyl-1,2,3a,4,5,9b-hexahydro-3H-benz[e]indol, (+)-3aS,5S,9bR-3-Methyl-5-phenyl-1,2,3a,4,5,9b-hexahydro-3H-benz[e]indol, (±)-3aS(R),5S(R),9bR(S)-3-Ethyl-5-phenyl-1,2,3a,4,5,9b-hexahydro-3H-benz[e]indol und (±)-3aS(R),5S(R),9bR(S)-3-n-Propyl-5-phenyl-1,2,3a,4,5,9b-hexahydro-3H-benz[e]indol.

Die einzelnen Diastereomeren lassen sich mit Hilfe üblicher Methoden zur Racematspaltung über die Salze mit chiralen Carbonsäuren in ihre Antipoden trennen.

Die absolute Konfiguration der chiralen Zentren in I läßt sich mit Hilfe von Röntgenstrukturanalyse ermitteln.

Die Verbindungen der Formel I lassen sich herstellen, indem man

a) ein 3-Phenyl-2-styryl-pyrrolidin der Formel II

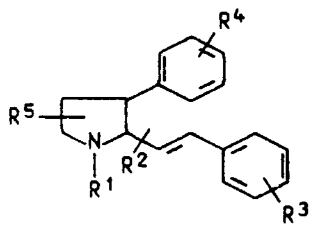

II

in der $R^1$-$R^5$ die in Anspruch 1 angegebene Bedeutung besitzen, in Gegenwart einer starken Säure cyclisiert oder

b) eine Verbindung der Formel III

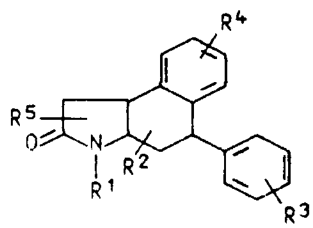

III,

worin $R^1$-$R^5$ die oben angegebene Bedeutung haben, reduziert

und anschließend gegebenenfalls in ihre Salze mit physiologisch verträglichen Säuren überführt.

Der Substituent $R^2$ steht - in Übereinstimmung mit Formel I - in den Verbindungen der Formel II entweder am C-2 des Pyrrolidinringes oder an dem mit diesem verbundenen C-Atom des Styrolrestes.

Als Säuren eignen sich für die Ringschlußreaktion a) starke Mineralsäuren, wie Salzsäure, Phosphorsäure und insbesondere Polyphosphorsäure und konzentrierte Schwefelsäure. Zweckmäßigerweise wird die Säure auch als Lösungsmittel verwendet. Die Cyclisierung gelingt bei Temperaturen von 0 bis 50 °C und ist in der Regel nach 1 bis 10 h beendet.

Setzt man bei dieser Cyclisierungsreaktion ein cis-3-Phenyl-2-styryl-Pyrrolidin-Derivat IIa ein, so erhält man selektiv die besonders wirksamen Diastereomeren Ia mit cis-Pyrrolidin-Anellierung. Liegt zudem die Doppelbindung im cis-Pyrrolidin-Derivat IIa noch in der stabileren trans-Konfiguration vor, so gelangt man selektiv in die wirksame 3aS(R),5S(R),9bR(S)-Reihe.

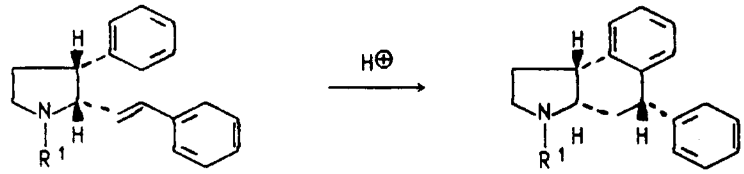


Ia

IIa

Die Reduktion der Verbindungen III wird mit komplexen Metallhydriden, wie z.B. Lithiumaluminiumhydrid, in einen inerten Lösungsmittel, vorzugsweise in Tetrahydrofuran, bei Temperaturen zwischen 0 und 80 °C durchgeführt.

Die Verbindungen der Formel I werden in der Mehrzahl der Fälle in Form von Kristallen erhalten und können durch Umkristallisation aus den üblichen organischen Lösungsmitteln, bevorzugt aus einem niederen Alkohol, wie Ethanol oder einem niedrigen Ester, vorzugsweise Essigsäureethylester, umkristallisiert oder durch Säulenchromatographie gereinigt werden.

Dabei können die eventuell anfallenden Diastereomerengemische der Formel I in die reinen Diastereomeren mit cis- bzw. trans-Anellierung des Pyrrolidins aufgetrennt werden, vorzugsweise durch fraktionierte Kristallisation aus einem niederen Alkohol.

Die reinen Diastereomeren lassen sich nach bekannten Methoden in die entsprechenden Antipoden

aufspalten, z.B. durch Bildung diastereomerer Salze mit Hilfe optisch aktiver Säuren. Beispielsweise löst man die freie Base der Formel I in einem niederen Alkohol und gibt 1 Äquivalent (+)- oder (-)-Weinsäure, (+)- oder (-)-Dibenzoyl-Weinsäure oder (+)- bzw. (-)-Di-p-toluoyl-Weinsäure hinzu. Die fraktionierte Kristallisation liefert dann zunächst das schwerer lösliche der beiden diastereomeren Salze, das bis zum konstanten Drehwert umkristallisiert wird. Nach Freisetzung der freien Base erhält man die enantiomerenreine Form der Verbindung I.

Die Ausgangsverbindungen der Formel II lassen sich durch Umsetzung von Zimtaldehyd oder Benzalaceton bzw. ihrer Derivate mit Monoalkylaminen und Wasserstoff in Gegenwart von Hydrierkatalysatoren herstellen:

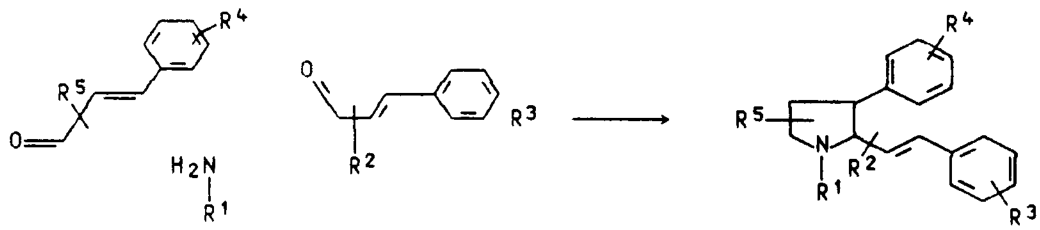

Die Umsetzung erfolgt in einem Autoklaven bei Temperaturen zwischen 70 und 150° C. In dem Autoklaven wird in einem Lösungsmittel suspendiert der Katalysator und nach Verdrängen der Luft das Monoalkylamin eingepreßt. Nach Einstellen der Reaktionstemperatur wird Wasserstoff aufgepreßt und die ungesättigte Carbonylverbindung in den Reaktionsraum zugepumpt.

Nach Aufnahme der theoretisch berechneten Wasserstoffmenge wird überschüssiges Amin entfernt, der Katalysator abgesaugt und das Reaktionsgemisch fraktioniert destilliert. Dabei erhält man im hochsiedenden Bereich die gewünschte Verbindung II, die in der Regel als Diastereomerengemisch anfällt.

Als Katalysator für die Umsetzung der $\alpha,\beta$-ungesättigten Carbonylverbindungen wurde ein Raney-Kobalt verwendet, dessen Reaktivität durch 200-600stündige Lagerung unter Wasser bei Temperaturen von 70 bis 90° C abgesenkt worden war. Wird die Inaktivierung des Katalysators zu weit getrieben, dann entstehen bei der Hydrierung in erster Linie höhersiedende Kondensationsprodukte. Ist der Katalysator zu aktiv, werden insbesondere die regulären Reaktionsprodukte der aminierenden Hydrierung erhalten.

Die Ausgangsverbindungen der Formel II lassen sich ferner aus 1-Alkyl-4-phenyl-5-formyl-pyrrolidin-2-on-Derivaten (DE-OS 35 37 075, 36 24 102, 36 32 589) durch Wittig-Reaktion mit Benzylphosphonium-Salzen bzw. Benzylphosphonaten in Gegenwart von Basen und gegebenenfalls anschließende Reduktion mit komplexen Metallhydriden, wie z.B. Lithiumaluminiumhydrid, in einem inerten organischen Lösungsmittel, vorzugsweise in Tetrahydrofuran, bei Temperaturen zwischen 0 und 80° C herstellen. Dasselbe gilt für die Herstellung der Ausgangsverbindungen der Formel III, wobei nach der Wittig-Reaktion die säurekatalysierte Cyclisierung durchgeführt wird.

Die freien 5-Phenyl-1,2,3a,4,5,9b-hexahydro-3H-benz[e]indole der Formel I können in üblicher Weise in das Säureadditionssalz einer pharmakologisch verträglichen Säure überführt werden, vorzugsweise durch Versetzen einer Lösung mit einem Äquivalent der entsprechenden Säure. Pharmazeutisch verträgliche Säuren sind beispielsweise Salzsäure, Phosphorsäure, Schwefelsäure, Methansulfonsäure, Amidosulfonsäure, Maleinsäure, Fumarsäure, Oxalsäure, Weinsäure und Zitronensäure.

Die erfindungsgemäßen Verbindungen weisen wertvolle pharmakologische Eigenschaften auf. Sie eignen sich beispielsweise zur Behandlung von psychischen Störungen, insbesondere von Depressionen, sowie als Sedativa und Tranquilizer und überraschenderweise auch aufgrund einer antikonvulsiven Wirkung gegen Krampfleiden.

Die antidepressive Wirkung der erfindungsgemäßen Substanzen wurde mit folgendem Modell untersucht:

2,15 mg/kg Reserpin, das männlichen Mäusen (NMRI-Stamm) mit einem Gewicht von 20 - 26 g subkutan verabreicht wurde, senkt die Körpertemperatur um durchschnittlich 3° C, gemessen zwei Stunden nach der Reserpinverabreichung und bei einer Umgebungstemperatur von 20 - 22° C. Antidepressiva hemmen diese Hypothermie, wobei die Hemmung von der Dosis abhängt. Die Testsubstanzen werden oral verabreicht, und zwar 60 Minuten vor der Reserpinverabreichung.

Die Dosis, die die reserpininduzierte Hypothermie um 50% hemmt, d.h. ED 50, wird bestimmt aus der linearen Regression zwischen log Dosis (mg/kg) und der relativen Abnahme der Hypothermie.

Die antikonvulsive Wirkung wurde an weiblichen Mäusen (NMRI-Stamm) mit einem Gewicht von 22 - 26

g gemessen. Tonische Krämpfe wurden durch Elektroschock über Ohrelektroden induziert. Reizdaten: Serie rechteckiger Impulse von 4,64 ms Dauer und 14,7 mA Amplitude; Frequenz 100 Hz; Dauer der Serie 0,2 sec.

Der Elektroschock wurde 60 min nach der oralen Verabreichung der Testverbindungen gegeben, und das Auftreten von tonischen Krämpfen wurde gezählt. n/Dosis = 8. Die Verhältnisse zwischen Wirkung (% geschützte Tiere) und log Dosis wurden durch Probit-Analyse errechnet. Die ED 50 ist die Dosis, die tonische Krämpfe bei 50% der Tiere hemmt.

Die ED-50-Werte (Tabelle 1) der erfindungsgemäßen Verbindungen liegen überwiegend unter dem für das Standard-Antidepressivum Imipramin ermittelten ED-50-Wert; die Substanzen haben also eine größere Wirkstärke als die Vergleichssubstanz. In einigen Fällen ist die Wirkstärke erheblich höher als die von Imipramin (z.B. Nr. 1 oder 4, um den Faktor von etwa 45).

Weiterhin besitzen die Substanzen antikonvulsive Eigenschaften (Tabelle 2). Die Wirkung von Phenytoin, einem klinisch bewährten Antiepileptikum, wird erreicht bzw. deutlich übertroffen (Beispiel 1, 6).

Die Verbindung von antidepressiven mit antikonvulsiven Eigenschaften stellt eine neue Wirkungskombination dar, da bisher bekannte tricyclische Antidepressiva nicht antikonvulsiv, sondern krampfverstärkend wirken können.

Tabelle 1

| Substanz aus Beispiel Nr. | Antidepressive Wirkung ED 50 mg/kg, oral verabreicht |
|---|---|
| 1 | 0,082 |
| 2 | 5,01 |
| 4 | 0,087 |
| 5 | 0,14 |
| 6 | 1,61 |
| 7 | 0,26 |
| Imipramin | 3,82 |

Tabelle 2

| Substanz aus Beispiel Nr. | Antikonvulsive Wirkung ED 50 mg/kg, oral verabreicht |
|---|---|
| 1 | 1,1 |
| 4 | ~46,4 |
| 5 | ~100 |
| 6 | 8,81 |
| 7 | 11,9 |
| Phenytoin | 14,3 |

Die Erfindung betrifft dementsprechend auch ein therapeutisches Mittel, gekennzeichnet durch einen Gehalt an einer Verbindung der Formel I oder deren pharmakologisch verträgliches Säureadditionssalz als Wirkstoff neben üblichen Träger- und Verdünnungsmitteln, sowie die Verwendung der neuen Verbindungen bei der Bekämpfung von Krankheiten.

Die erfindungsgemäßen Verbindungen können in üblicher Weise oral oder parenteral, intravenös oder intramuskulär verabfolgt werden.

Die Dosierung hängt vom Alter, Zustand und Gewicht des Patienten sowie von der Applikationsart ab. In der Regel beträgt die tägliche Wirkstoffdosis zwischen etwa 1 und 20 mg/kg Körpergewicht bei oraler Gabe und zwischen 0,1 und 2 mg/kg Körpergewicht bei parenteraler Gabe.

Die neuen Verbindungen können in gebräuchlichen galenischen Applikationsformen fest oder flüssig angewendet werden, z.B. als Tabletten, Filmtabletten, Kapseln, Pulver, Granulate, Dragees, Suppositorien, Lösungen, Salben, Cremes oder Sprays. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fließregulierungsmitteln, Weichmachern, Netzmitteln, Dispergiermitteln, Emulgatoren, Lösungsmitteln, Retardierungsmitteln, Antioxidantien und/oder Treibgasen verarbeitet werden (vgl` H. Sucker et al: Pharmazeutische Technologie, Thieme-Verlag, Stuttgart, 1978). Die so erhaltenen Applikationsformen enthalten den Wirkstoff normalerweise in einer Menge von 0,1 bis 99 Gew.%.

Die folgenden Beispiele erläutern die Erfindung.

A. Herstellung der Ausgangsstoffe

a) 3-Phenyl-2-trans-styryl-N-methyl-pyrrolidin

In einem 10 l-Rührautoklaven wurden 1,4 kg Monomethylamin, 1,6 kg Ethanol und 0,1 kg Raney-Kobalt gegeben. Der Autoklav wurde auf 100 ° C erhitzt und unter einem Druck von 150 bar Wasserstoff zugeführt. Im Verlauf von 10 h wurden 1,6 kg Zimtaldehyd zugepumpt. Nach Ende der Wasserstoffaufnahme wurde das Reaktionsgemisch nach dem Abkühlen filtriert und fraktioniert destilliert. Die bei 153 bis 184 ° C/2 mbar übergehende Fraktion enthielt 99,5 g cis-3-Phenyl-2-trans-styryl-N-methyl-pyrrolidin und 68,5 g trans-3-Phenyl-2-trans-styryl-N-methyl-pyrrolidin.

Das so erhaltene Diastereomerengemisch wurde durch fraktionierte Destillation über eine Füllkörperkolonne mit einer Edelstahldrahtnetzwendel (Länge 160 cm) getrennt. Das cis-Diastereomere ging bei 157 ° C/2 mbar über und war zu über 95 % rein, das trans-Diastereomere ging bei 160-161 ° C/2 mbar über und war zu über 90 % rein.

Beim Stehen kristallisiert das trans-Diastereomere und kann aus Essigester oder n-Octan umkristallisiert werden, Schmp: 63-64 ° C.

b) 3-Phenyl-2-trans-styryl-N-ethyl-pyrrolidin

In einem 10 l-Autoklaven wurden 2,0 kg Zimtaldehyd mit 1,4 kg Ethylamin bei 90 ° C unter einem Wasserstoffdruck von 150 bar in Gegenwart von 100 g Raney-Kobalt in 1,6 kg Ethanol umgesetzt. Die Zeit für das Zupumpen des Zimtaldehyds betrug 10 h. Nach Zugabe des Zimtaldehyds wurden die Reaktionsbedingungen noch 6 h aufrechterhalten.

Das Reaktionsgemisch wurde anschließend fraktioniert destilliert. Bei 160-165 ° C/2 mbar wurde eine Fraktion erhalten, die 141 g cis- und 161 g trans-3-Phenyl-2-trans-styryl-N-ethyl-pyrrolidin enthielt.

c) 3-Phenyl-2-trans-styryl-N-n-propyl-pyrrolidin

In einem 10 l-Autoklaven wurden 2 kg Zimtaldehyd mit 1,6 kg n-Propylamin bei 90 ° C unter einem Wasserstoffdruck von 150 bar in Gegenwart von 100 g Raney-Kobalt umgesetzt. Die Zeit für das Zupumpen des Zimtaldehyds betrug 10 h. Das Reaktionsgemisch wurde fraktioniert destilliert. Die bei 150-175 ° C/2 mbar übergehende Fraktion wurde analog a) fraktioniert destilliert. Man erhielt bei 164-166 ° C/2 mbar 130 g cis-3-Phenyl-2-trans-styryl-N-n-propyl-pyrrolidin (85 % rein) und bei 167-168 ° C/2 mbar 145 g trans-3-Phenyl-2-trans-styryl-N-n-propyl-pyrrolidin (94 % rein).

In analoger Weise wurden die substituierten 3-Phenyl-2-trans-styryl-pyrrolidin-Derivate der Formel II durch Einsatz von substituierten Zimtaldehyd- bzw. Benzalaceton-Derivaten in die aminierende Hydrierung hergestellt.

d) cis-1-Methyl-4-phenyl-5-m-chlorstyryl-pyrrolidin-2-on

Zu 36,0 g (77 mM) Triphenyl-m-chlor-benzyl-phosphonium-bromid in 150 ml Toluol tropfte man unter Eiskühlung und Stickstoffschutzgas innerhalb 20 min 32,8 g (77 mM) n-Butyllithium (15 % in Hexan) und ließ die orangefarbene Suspension noch 15 min nachrühren. Anschließend tropfte man 15,6 g (77 mM) cis-5-Formyl-1-methyl-4-phenyl-pyrrolidin-2-on (DE-OS 35 37 075, 36 32 589; J. Org. Chem. 52, 4352 (1987)) in 120 ml Toluol hinzu und ließ 3 h bei Raumtemperatur nachrühren (Entfärbung nach hellgelb). Man fügte zum Ansatz 200 ml $H_2O$ hinzu, säuerte mit verdünnter HCl an, trennte nach Absaugen die Phasen, extrahierte die wäßrige Phase noch zweimal mit Toluol, wusch die vereinigten organischen Phasen mit $H_2O$, trocknete und engte ein. Das Rohprodukt wurde in 100 ml Methyl-t-butylether aufgenommen und unter Kühlen vom ausgefallenen Triphenylphosphinoxid abgesaugt. Das Filtrat wurde eingeengt und durch Säulenchromatographie (Kieselgel, Laufmittel Methylenchlorid/Methanol 98/2) gereinigt; Ausbeute: 16,1 g (67 %). Die Styryl-Seitenkette liegt nach dem $^1$H-NMR-Spektrum zu 90 % in der trans- und zu 10 % in der cis-Konfiguration vor.

Bei Einsatz von am Benzolkern substituierten 5-Formyl-1-methyl-4-phenyl-pyrrolidin-2-on-Derivaten erhält man in analoger Weise die entsprechenden am 4-Phenylkern substituierten Verbindungen (DE-OS 35 37 075, 36 32 589, J. Org. Chem. 52, 4352 (1987)).

e) cis-1-Methyl-3-phenyl-2-trans-m-chlorstyryl-pyrrolidin

Zu 0,65 g (17 mM) Lithiumaluminiumhydrid in 30 ml Ether tropfte man bei 0 bis 5 ° C unter Stickstoff eine Lösung aus 3,0 g (9,6 mM) cis-1-Methyl-4-phenyl-5-m-chlorstyryl-pyrrolidin-2-on in 40 ml Ether hinzu und ließ 1 h bei Eiskühlung nachrühren. Anschließend wurde unter Kühlung 10 %ige Natronlauge langsam zugetropft, bis der Niederschlag an der Gefäßwand konglomerierte. Die überstehende Etherpha-

se wurde mit $H_2O$ gewaschen (pH = 10), getrocknet und eingeengt. Reinigung durch Säulenchromatographie (Kieselgel, Laufmittel Methylenchlorid/Methanol 95/5) lieferte die freie Base (2,3 g, 80 %). Diese wurde in Essigester aufgenommen und mit etherischer HCl in das Hydrochlorid überführt; Schmp.: 53-56 ° C.

B. Herstellung der Endprodukte

Beispiel 1

(±)-3aS(R),5S(R),9bR(S)-3-Methyl-5-phenyl-1,2,3a,4,5,9b-hexahydro-3H-benz[e]indol-hydrochlorid

100,0 g (380 mM) cis-3-Phenyl-2-trans-styryl-N-methyl-pyrrolidin in 235 ml Methylenchlorid ließ man langsam zu einer intensiv gerührten Mischung aus 108 ml konzentrierter Schwefelsäure und 493 ml Methylenchlorid bei 0 bis 5 ° C zutropfen. Der Ansatz wurde anschließend noch 1 h bei 0 bis 5 ° C und dann über Nacht bei Raumtemperatur gerührt. Zur Aufarbeitung goß man auf 2,5 l Eis/Wasser, stellte mit 10 proz. Natronlauge auf pH = 8, trennte die organische Phase ab und extrahierte die wäßrige Phase noch dreimal mit Methylenchlorid. Die vereinigten organischen Phasen wurden mit Wasser gewaschen, getrocknet und eingeengt. Den Rückstand nahm man in Diisopropylether auf, filtrierte die unlöslichen Verunreinigungen ab und engte das Filtrat zur Trockene ein.

Das verbleibende Öl (96,2 g) löste man in 200 ml Ethanol, überführte mit etherischer Salzsäure in das Hydrochlorid, engte die Lösung auf 150 ml ein und gab bis zur auftretenden leichten Trübung Ether hinzu. Die über Nacht gebildeten, leicht hygroskopischen Kristalle wurden unter Stickstoff abgesaugt und im Vakuum getrocknet.

Die Mutterlauge wurde auf die Hälfte eingeengt, wieder bis zur beginnenden Trübung mit Ether versetzt und das Kristallisat abgesaugt. Durch nachfolgende zweimalige Wiederholung dieser Operation und Vereinigung der Kristallisate isolierte man zusammen 83,5 g (74 %) Produkt mit dem Schmp. 222-223 ° C.

Die Röntgenstrukturanalyse ergab die 3aS(R),5S(R),9bR(S)-Konfiguration der chiralen Zentren (cis-Pyrrolidin-Anellierung).

Zur Überführung in die freie Base suspendierte man das Hydrochlorid in Wasser, machte mit Natronlauge alkalisch und extrahierte mit Methylenchlorid. Schmp. der freien Base: 63-64 ° C.

Beispiel 2

(±)-3aR(S),5R(S),9bR(S)-3-Methyl-5-phenyl-1,2,3a,4,5,9b-hexahydro-3H-benz[e]indol

Die Synthese erfolgte analog Beispiel 1 unter Einsatz von trans-3-Phenyl-2-trans-styryl-N-methyl-pyrrolidin. Das nach dem Einengen der organischen Phase verbliebene Rohprodukt wurde als freie Base aus Diisopropylether umkristallisiert. Ausbeute 71 %, farblose Nadeln, Schmp. 96-98 ° C.

Die Röntgenstrukturanalyse ergab die 3aR(S),5R(S),9bR(S)-Konfiguration der chiralen Zentren (trans-Pyrrolidin-Anellierung)

Beispiel 3

(±)-3aS(R),5S(R),9bR(S)-3-Methyl-5-phenyl-1,2,3a,4,5,9b-hexahydro-3H-benz[e]indol-hydrochlorid und dessen Diastereomeres

Die Synthese erfolgte analog Beispiel 2 unter Einsatz von cis,trans-3-Phenyl-2-trans-styryl-N-methyl-pyrrolidin. Die Kristallisation des Rohprodukts aus Diisopropylether lieferte als schwerer lösliche Fraktion praktisch reines (±)-3aR(S),5R(S),9bR(S)-3-Methyl-5-phenyl-1,2,3a,4,5,9b-hexahydro-3H-benz[e]indol (vgl. Beispiel 2); Schmp.: 94-96 ° C.

Die Mutterlauge wurde mit etherischer Salzsäure versetzt, bis kein Niederschlag mehr ausfiel. Das Reaktionsgemisch wurde auf 30-40 ° C erwärmt und mit Ethanol versetzt, bis eine klare Lösung entstanden war. Anschließend wurde Ether zugesetzt, bis eine leichte Trübung auftrat. Nach Abkühlen des Gemisches kristallisierte (±)-3aS(R),5S(R),9bR(S)-3-Methyl-5-phenyl-1,2,3a,4,5,9b-hexahydro-3H-benz[e]indol-hydrochlorid, Schmp.:49-51 ° C, aus.

Beispiel 4

(+)-3aS,5S,9bR-3-Methyl-5-phenyl-1,2,3a,4,5,9b-hexahydro-3H-benz[e]indol

20,0 g (76 mM) (±)-3aS(R),5S(R),9bR(S)-3-Methyl-5-phenyl-1,2,3a,4,5,9b-hexahydro-3H-benz[e]indol (vgl. Beispiel 1) in 300 ml Ethanol wurden mit einer Lösung von 29,4 g (76 mM) (-)-Di-O-p-toluoyl-L-Weinsäure in 300 ml Ethanol versetzt. Die über Nacht gebildeten Kristalle wurden unter Nachwaschen mit Ethanol abgesaugt und noch zweimal aus Ethanol umkristallisiert: $[\alpha]_D$ = -6,9° (DMF, c=1,007). Zur Überführung des Salzes in die optisch reine freie Base verteilte man das Salz zwischen Methylenchlorid und 10 %iger Natronlauge und isolierte nach dem üblichen Aufarbeiten und Umkristallisieren aus Ethanol 7,8 g (39 %) (+)-3aS,5S,9bR-3-Methyl-5-phenyl-1,2,3a,4,5,9b-hexahydro-3H-benz[e]indol, $[\alpha]_D$ = + 58,3° (Ethanol, c=0,980), Schmp. 92°C. Die Röntgenstrukturanalyse ergab die angegebene Konfiguration. Schmp. des Hydrochlorids: 238-240°C.

Analog Beispiel 1 bis 3 wurden hergestellt:

5. (±)-3aS(R),5S(R),9bR(S)-3-Ethyl-5-phenyl-1,2,3a,4,5,9b-hexahydro-3H-benz[e]indol-hydrochlorid, Schmp. 237-239°C (Ausgangsmaterial: cis-3-Phenyl-2-trans-styryl-N-ethyl-pyrrolidin),
6. (±)-3aR(S),5R(S),9bR(S)-3-Ethyl-5-phenyl-1,2,3a,4,5,9b-hexahydro-3H-benz[e]indol-hydrochlorid, Schmp. 234-236°C (Ausgangsmaterial: trans-3-Phenyl-2-trans-styryl-N-ethyl-pyrrolidin),
7. (±)-3aS(R),5S(R),9bR(S)-3-n-Propyl-5-phenyl-1,2,3a,4,5,9b-hexahydro-3H-benz[e]indol-hydrochlorid, Schmp. 255-257°C (Ausgangsmaterial: cis-3-Phenyl-2-trans-styryl-N-n-propyl-pyrrolidin),
8. (±)-3aR(S),5R(S),9bR(S)-3-n-Propyl-5-phenyl-1,2,3a,4,5,9b-hexahydro-3H-benz[e]indol-hydrochlorid, Schmp. >275°C (Ausgangsmaterial: trans-3-Phenyl-2-trans-styryl-N-n-propyl-pyrrolidin),

Beispiel 9

(±)-2,3,3a-Trimethyl-5-phenyl-1,2,3a,4,5,9b-hexahydro-3H-benz[e]indol-hydrochlorid

3,5 g (12,0 mM) 1,2,5-Trimethyl-3-phenyl-2-trans-styryl-pyrrolidin in 10 ml Methylenchlorid ließ man langsam zu einer intensiv gerührten Mischung aus 4,5 ml konzentrierter Schwefelsäure und 20 ml Methylenchlorid bei 0 bis 5°C zutropfen. Der Ansatz wurde anschließend noch 1 h bei 0 bis 5°C und dann über Nacht bei Raumtemperatur gerührt. Zur Aufarbeitung goß man auf 300 ml Eis/Wasser, stellte mit 10 %iger Natronlauge auf pH = 8, trennte die organische Phase ab und extrahierte die wäßrige Phase noch dreimal mit Methylenchlorid. Die vereinigten organischen Phasen wurden mit Wasser gewaschen, getrocknet und eingeengt.

Die Abtrennung zweier Diastereomerer durch Säulenchromatographie (Kieselgel, Methylenchlorid/Methanol 95/5) lieferte eine unpolare und eine polare Fraktion, die jeweils in Ether gelöst, filtriert und mit überschüssiger etherischer Salzsäure versetzt wurden. Die nach 15 min Rühren ausgefallenen farblosen Festkörper saugte man in der Kälte unter Stickstoff-Schutzgas ab und trocknete im Vakuumtrockenschrank.

Man isolierte die beiden diastereomeren Hydrochloride in einer Ausbeute von 1,6 bzw. 0,5 g (41 bzw. 13 %) mit den Schmelzpunkten 252-254°C bzw. 264-266°C.

Analog Beispiel 9 wurden hergestellt:

10. (±)-1,3,4-Trimethyl-5-phenyl-1,2,3a,4,5,9b-hexahydro-3H-benz[e]indol-hydrochlorid (Ausgangsmaterial: 1,4-Dimethyl-3-phenyl-2-trans-β-methyl-styryl-pyrrolidin).
11. (±)-1,4-Dimethyl-3-ethyl-5-phenyl-1,2,3a,4,5,9b-hexahydro-3H-benz[e]indol-hydrochlorid, Schmp. 243-246°C
12. (±)-2,3a-Dimethyl-3-ethyl-5-phenyl-1,2,3a,4,5,9b-hexahydro-3H-benz[e]indol-hydrochlorid, Schmp. 187-190°C
13. (±)-7-Chlor-3-methyl-5-phenyl-1,2,3a,4,5,9b-hexahydro-3H-benz[e]indol-hydrochlorid,
14. (±)-7-Chlor-3-ethyl-5-phenyl-1,2,3a,4,5,9b-hexahydro-3H-benz[e]indol-hydrochlorid,

15a (±)-3aS(R),5S(R),9bR(S)-3-Methyl-5-m-chlor-phenyl-1,2,3a,4,5,9b-hexahydro-3H-benz[e]indol-2-on

7,5 g (24 mM) cis-1-Methyl-4-phenyl-5-m-chlorstyryl-pyrrolidin-2-on in 70 g Polyphosphorsäure wurden bei 100°C 1 h lang gerührt. Den Ansatz goß man anschließend auf 0,5 l Eiswasser, machte mit konzentrierter Natronlauge alkalisch und extrahierte die wäßrige Phase zweimal mit Methylenchlorid. Die vereinigten organischen Phasen wurden mit Wasser gewaschen, getrocknet und eingeengt. Den Rückstand löste man in wenig Essigester, setzte die dreifache Volumenmenge Cyclohexan hinzu und ließ

unter Kühlung auskristallisieren. Die Kristalle wurden abgesaugt, mit Cyclohexan nachgewaschen und im Vakuum getrocknet. Man erhielt 5,9 g (79 %); Schmp.: 147-148 ° C.

15b (±)-3aS(R),5S(R),9bR(S)-3-Methyl-5-m-chlor-phenyl-1,2,3a,4,5,9b-hexahydro-3H-benz[e]indol-hydrochlorid

Zu 1,78 g (47 mM) Lithiumaluminiumhydrid in 20 ml Tetrahydrofuran tropfte man bei 0 bis 5 ° C unter Stickstoff eine Lösung von 3,8 g (12 mM) (±)-3aS(R),5S(R),9bR(S)-3-Methyl-5-m-chlor-phenyl-1,2,3a,4,5,9b-hexahydro-3H-benz[e]indol-2-on in 80 ml Tetrahydrofuran hinzu und ließ 3 h unter Rückflußkochen nachrühren. Anschließend wurde unter Kühlung 10 %ige Natronlauge langsam zugetropft, bis der Niederschlag an der Gefäßwand konglomerierte. Nach dessen Absaugen wurde die organische Phase mit Wasser gewaschen (pH = 10), getrocknet und eingeengt. Das zurückbleibende gelbe Öl nahm man in 120 ml Ether auf, setzte unter Kühlung und Stickstoff etherische Salzsäure hinzu, saugte die ausgefallenen Festkörper unter Stickstoff ab, wusch gut mit Ether nach und trocknete das Produkt im Vakuumtrockenschrank. Man erhielt 3,4 g (85 %); Schmp.: 75-78 ° C.

Analog Beispiel 15 wurden hergestellt:

16. (±)-3aS(R),5S(R),9bR(S)-3-Methyl-5-p-chlor-phenyl-1,2,3a,4,5,9b-hexahydro-3H-benz[e]indol-hydrochlorid,
17. (±)-3aS(R),5S(R),9bR(S)-3-Methyl-5-p-methyl-phenyl-1,2,3a,4,5,9b-hexahydro-3H-benz[e]indol-hydrochlorid,
18. (±)-3aS(R),5S(R),9bR(S)-3-Methyl-5-m-methoxy-phenyl-1,2,3a,4,5,9b-hexahydro-3H-benz[e]indol-hydrochlorid,
19. (±)-3aS(R),5S(R),9bR(S)-3-Methyl-5-p-trifluormethyl-phenyl-1,2,3a,4,5,9b-hexahydro-3H-benz[e]indol-hydrochlorid,
20. (±)-3aS(R),5S(R),9bR(S)-3-Methyl-5-m-fluor-phenyl-1,2,3a,4,5,9b-hexahydro-3H-benz[e]indol-hydrochlorid,
21. (±)-3aS(R),5S(R),9bR(S)-3-Methyl-5-o-chlor-phenyl-1,2,3a,4,5,9b-hexahydro-3H-benz[e]indol-hydrochlorid,
22. (±)-3aS(R),5S(R),9bR(S)-3-Methyl-5-(2,4-dichlor-phenyl)-1,2,3a,4,5,9b-hexahydro-3H-benz[e]indol-hydrochlorid,
23. (±)-3aS(R),5S(R),9bR(S)-3-Methyl-5-p-fluor-phenyl-1,2,3a,4,5,9b-hexahydro-3H-benz[e]indol-hydrochlorid,
24. (±)-3aS(R),5S(R),9bR(S)-3-Methyl-5-m-methyl-phenyl-1,2,3a,4,5,9b-hexahydro-3H-benz[e]indol-hydrochlorid,
25. (±)-3aS(R),5S(R),9bR(S)-3-Methyl-5-o-fluor-phenyl-1,2,3a,4,5,9b-hexahydro-3H-benz[e]indol-hydrochlorid,
26. (±)-3aS(R),5S(R),9bR(S)-3-Methyl-5-p-thiomethyl-phenyl-1,2,3a,4,5,9b-hexahydro-3H-benz[e]indol-hydrochlorid,
27. (±)-3aS(R),5S(R),9bR(S)-3-Methyl-5-p-methoxy-phenyl-1,2,3a,4,5,9b-hexahydro-3H-benz[e]indol-hydrochlorid,
28. (±)-3aS(R),5S(R),9bR(S)-3-Methyl-5-p-hydroxy-phenyl-1,2,3a,4,5,9b-hexahydro-3H-benz[e]indol-hydrochlorid (Herstellung aus 28 durch Etherspaltung mit Bortribromid in Chloroform).
29. (±)-3aS(R),5S(R),9bR(S)-3-Methyl-5-p-acetamino-phenyl-1,2,3a,4,5,9b-hexahydro-3H-benz[e]indol-hydrochlorid,
30. (±)-3aS(R),5S(R),9bR(S)-3-Methyl-5-p-amino-phenyl-1,2,3a,4,5,9b-hexahydro-3H-benz[e]indol-hydrochlorid.

Beispiel 31

Auf einer Tablettenpresse werden in üblicher Weise Tabletten folgender Zusammensetzung gepreßt:

| | |
|---|---|
| 12,5 mg | Substanz des Beispiels 1 |
| 120 mg | Maisstärke |
| 13,5 mg | Gelatine |
| 45 mg | Milchzucker |
| 2,25 mg | Aerosil® (chemisch reine Kieselsäure in submikroskopisch feiner Verteilung) |
| 6,75 mg | Kartoffelstärke (als 6 %iger Kleister) |

Beispiel 32

In üblicher Weise werden Dragees folgender Zusammensetzung hergestellt:

| | |
|---|---|
| 7,5 mg | Substanz des Beispiels 5 |
| 60 mg | Kernmasse |
| 60 mg | Verzuckerungsmasse |

Die Kernmasse besteht aus 9 Teilen Maisstärke, 3 Teilen Milchzucker und 1 Teil Luviskol® VA 64 (Vinylpyrrolidon-Vinylacetat-Mischpolymerisat 60:40, vgl. Pharm.Ind. 1962, 586). Die Verzuckerungsmasse besteht aus 5 Teilen Rohrzucker, 2 Teilen Maisstärke, 2 Teilen Calciumcarbonat und 1 Teil Talk. Die so hergestellten Dragees werden anschließend mit einem magensaftresistenten Überzug versehen.

Beispiel 33

1 g Substanz des Beispiels 7 wird in 5000 ml Wasser unter Zusatz von NaCl gelöst und mit 0,1 N NaOH auf oH 6,0 eingestellt, so daß eine blutisotonische Lösung entsteht. Jeweils 5 ml dieser Lösung werden in Ampullen gefüllt und sterilisiert.

**Patentansprüche**
**Patentansprüche für folgende Vertragsstaaten : BE, CH, DE, FR, GB, IT, NL**

**1.** 5-Phenyl-1,2,3a,4,5,9b-hexahydro-3H-benz[e]indole der Formel I

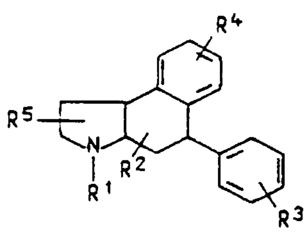

I,

in der

| | |
|---|---|
| $R^1$ | Wasserstoff oder $C_{1-6}$-Alkyl |
| $R^2$ | Wasserstoff oder $C_{1-3}$-Alkyl in der 3a- oder 4-Stellung |
| $R^3$ und $R^4$ | Wasserstoff, Hydroxy, Halogen, $C_{1-3}$-Alkyl, $C_{1-3}$-Alkoxy, $C_{1-3}$-Alkylthio oder Trifluormethyl, Acetamino, Amino und |
| $R^5$ | Wasserstoff oder $C_{1-3}$-Alkyl |

bedeuten und ihre Salze mit physiologisch verträglichen Säuren.

**2.** Verbindungen der Formel I nach Anspruch 1, in denen

| | |
|---|---|
| $R^1$ | Wasserstoff, Methyl, Ethyl oder n-Propyl, |
| $R^2$, $R^4$ und $R^5$ | Wasserstoff und |
| $R^3$ | Wasserstoff, Fluor, Chlor, Trifluormethyl, Methoxy, Thiomethyl, Methyl, Hydroxy, Acetamino oder Amino |

bedeuten.

**3.** (±)-3aS(R),5S(R),9bR(S)-3-Methyl-5-phenyl-1,2,3a,4,5,9b-hexahydro-3H-benz[e]indol gemäß Anspruch 1.

**4.** ( + )-3aS,5S,9bR-3-Methyl-5-phenyl-1,2,3a,4,5,9b-hexahydro-3H-benz[e]-indol gemäß Anspruch 1.

**5.** (±)-3aS(R),55(R),9bR(S)-3-Ethyl-5-phenyl-1,2,3a,4,5,9b-hexahydro-3H-benz[e]indol gemäß Anspruch 1.

**6.** (±)-3aS(R),55(R),9bR(S)-3-n-Propyl-5-phenyl-1,2,3a,4,5,9b-hexahydro-3H-benz[e]indol gemäß Anspruch 1.

**7.** (±)-3aS(R),5S(R),9bR(S)-3-Methyl-5-m-chlor-phenyl-1,2,3a,4,5,9b-hexahydro-3H-benz(e)indol gemäß Anspruch 1.

8. (±)-3aS(R),5S(R),9bR(S)-3-Methy-5-m-fluor-pnenyl-1,2,3a,4,5,9b-hexahydro-3H-benz(e)indol gemäß Anspruch 1.

9. Therapeutisches Mittel, enthaltend eine Verbindung der Formel I gemäß Anspruch 1.

10. Verbindungen der Formel I gemäß Anspruch 1 zur Anwendung bei der Bekämpfung von Krankheiten.

**Patentansprüche für folgende Vertragsstaaten : AT, ES**

1. Verfahren zur Herstellung der 5-Phenyl-1,2,3a,4,5,9b-hexahydro-3H-benz[e]indole der Formel I

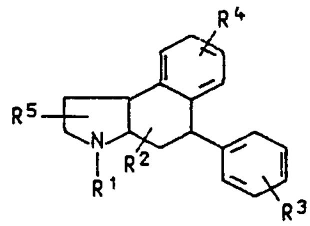

I,

in der

| | |
|---|---|
| $R^1$ | Wasserstoff oder $C_{1-6}$-Alkyl |
| $R^2$ | Wasserstoff oder $C_{1-3}$-Alkyl in der 3a- oder 4-Stellung |
| $R^3$ und $R^4$ | Wasserstoff, Hydroxy, Halogen, $C_{1-3}$-Alkyl, $C_{1-3}$-Alkoxy, $C_{1-3}$-Alkylthio oder Trifluormethyl, Acetamino, Amino und |
| $R^5$ | Wasserstoff oder $C_{1-3}$-Alkyl |

bedeuten, und deren Salze mit physiologisch verträglichen Säuren, bedeuten, dadurch gekennzeichnet, daß man

a) eine Verbindung der Formel II

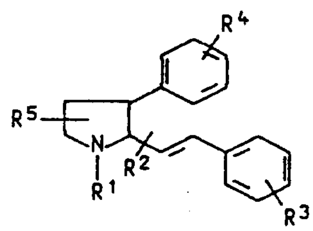

II,

in der $R^1$-$R^5$ die angegebene Bedeutung besitzen, in Gegenwart einer starken Säure cyclisiert oder

b) eine Verbindung der Formel III

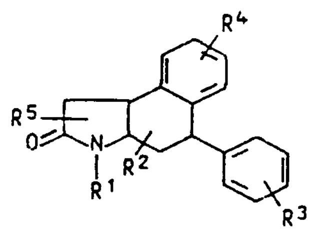

III,

worin $R^1$-$R^5$ die oben angegebene Bedeutung haben, reduziert

und anschließend die so erhaltenen Verbindungen gegebenenfalls in ihre Salze mit physiologisch verträglichen Säuren überführt,

2. Verfahren gemäß Anspruch 1, dadurch gekennzeichnet, daß man Verbindungen der Formel I herstellt, in denen

| | |
|---|---|
| $R^1$ | Wasserstoff, Methyl, Ethyl oder n-Propyl, |

| | |
|---|---|
| $R^2$, $R^4$ und $R^5$ | Wasserstoff und |
| $R^3$ | Wasserstoff, Fluor, Chlor, Trifluormethyl, Methoxy, Thiomethyl, Methyl, Hydroxy, Acetamino oder Amino |

bedeuten.

**3.** Verfahren gemäß Anspruch 1, dadurch gekennzeichnet, daß man (±)-3aS(R),5S(R),9bR(S)-3-Methyl-5-phenyl-1,2,3a,4,5,9b-hexahydro-3H-benz[e]indol herstellt.

**4.** Verfahren gemäß Anspruch 1, dadurch gekennzeichnet, daß man (±)-3aS,5S,9bR-3-Methyl-5-phenyl-1,2,3a,4,5,9b-hexahydro-3H-benz[e]indol herstellt.

**5.** Verfahren gemäß Anspruch 1, dadurch gekennzeichnet, daß man (±)-3aS(R),5S(R),9bR(S)-3-Ethyl-5-phenyl-1,2,3a,4,5,9b-hexahydro-3H-benz[e]indol herstellt.

**6.** Verfahren gemaß Anspruch 1, dadurch gekennzeichnet, daß man (±)-3aS(R),5S(R),9bR(S)-3-n-Propyl-5-phenyl-1,2,3a,4,5,9b-hexahydro-3H-benz[e]indol herstellt.

**7.** Verfahren gemäß Anspruch 1, dadurch gekennzeichnet, daß man (±)-3aS(R),5S(R),9bR(S)-3-Methyl-5-m-chlor-phenyl-1,2,3a,4,5,9b-hexahydro-3H-benz(e)indol herstellt.

**8.** Verfahren gemäß Anspruch 1, dadurch gekennzeichnet, daß man (±)-3aS(R),5S(R),9bR(S)-3-Methyl-5-fluor-phenyl-1,2,3a,4,5,9b-hexahydro-3H-benz(e)indol herstellt.

## Claims

## Claims for the following Contracting States : BE, CH, DE, FR, GB, IT, NL

**1.** A 5-phenyl-1,2,3a,4,5,9b-hexahydro-3H-benz[e]indole of the formula I

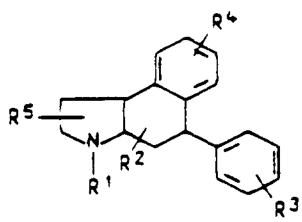

I,

where $R^1$ is hydrogen or $C_{1-6}$-alkyl, $R^2$ is hydrogen or $C_{1-3}$-alkyl in the 3a- or 4-position, $R^3$ and $R^4$ are each hydrogen, hydroxyl, halogen, $C_{1-3}$-alkyl, $C_{1-3}$-alkoxy, $C_{1-3}$-alkylthio or trifluoromethyl, acetamino or amino and $R^5$ is hydrogen or $C_{1-3}$-alkyl,
or a salt thereof with a physiologically tolerated acid.

**2.** A compound of the formula I as claimed in claim 1, where $R^1$ is hydrogen, methyl, ethyl or n-propyl, $R^2$, $R^4$ and $R^5$ are each hydrogen and $R^3$ is hydrogen, fluorine, chlorine, trifluoromethyl, methoxy, thiomethyl, methyl, hydroxyl, acetamino or amino.

**3.** (±)-3aS(R),5S(R),9bR(S)-3-Methyl-5-phenyl-1,2,3a,4,5,9b-hexahydro-3H-benz[e]indole as claimed in claim 1.

**4.** (±)-3aS,5S,9bR-3-Methyl-5-phenyl-1,2,3a,4,5,9b-hexahydro-3H-benz[e]indole as claimed in claim 1.

**5.** (±)-3aS(R),5S(R),9bR(S)-3-Ethyl-5-phenyl-1,2,3a, 4,5,9b-hexahydro-3H-benz[e]indole as claimed in claim 1.

**6.** (±)-3aS(R),5S(R),9bR(S)-3-n-Propyl-5-phenyl-1,2,3a,4,5,9b-hexahydro-3H-benz[e]indole as claimed in claim 1.

**7.** (±)-3aS(R),5S(R),9bR(S)-3-methyl-5-m-chlorophenyl-1,2,3a,4,5,9b-hexahydro-3H-benz[e]indole as claimed in claim 1.

**8.** (±)-3aS(R),5S(R),9bR(S)-3-methyl-5-m-fluorophenyl-1,2,3a,4,5,9b-hexahydro-3H-benz[e]indole as claimed in claim 1.

**9.** A therapeutic composition containing a compound of the formula 1 as claimed in claim 1.

**10.** A compound of the formula I as claimed in claim 1 for use in the treatment of disorders.

**Claims for the following Contracting States : AT, ES**

**1.** A process for preparing a 5-phenyl-1,2,3a,4,5,9b-hexahydro-3H-benz[e]indole of the formula I

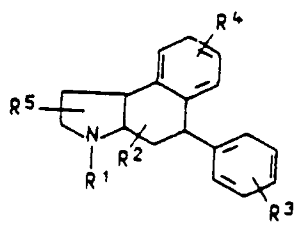

I,

where $R^1$ is hydrogen or $C_{1-6}$-alkyl, $R^2$ is hydrogen or $C_{1-3}$-alkyl in the 3a- or 4-position, $R^3$ and $R^4$ are each hydrogen, hydroxyl, halogen, $C_{1-3}$-alkyl, $C_{1-3}$-alkoxy, $C_{1-3}$-alkylthio or trifluoromethyl, acetamino or amino and $R^5$ is hydrogen or $C_{1-3}$-alkyl, or a salt thereof with a physiologically tolerated acid, which comprises

a) cyclizing a compound of the formula II

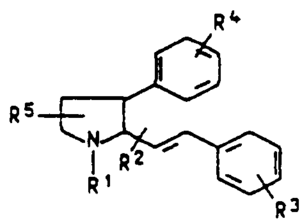

II,

where $R^1$-$R^5$ are each as defined above, in the presence of a strong acid or

b) reducing a compound of the formula III

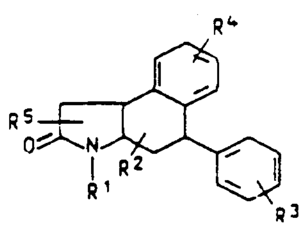

III,

where $R^1$-$R^5$ are each as defined above

and subsequently, if desired, converting the compound thus obtained into a salt thereof with a physiologically tolerated acid.

**2.** A process as claimed in claim 1, wherein a compound of the formula I is prepared, where $R^1$ is hydrogen, methyl, ethyl or n-propyl, $R^2$, $R^4$ and $R^5$ are each hydrogen and $R^3$ is hydrogen, fluorine, chlorine, trifluoromethyl, methoxy, thiomethyl, methyl, hydroxyl, acetamino or amino.

**3.** A process as claimed in claim 1, wherein (±)-3aS(R),5S(R),9bR(S)-3-methyl-5-phenyl-1,2,3a,4,5,9b-hexahydro-3H-benz[e]indole is prepared.

**4.** A process as claimed in claim 1, wherein (±)-3aS,5S,9bR-3-methyl-5-phenyl-1,2,3a,4,5,9b-hexahydro-3H-benz[e]indole is prepared.

5. A process as claimed in claim 1, wherein (±)-3aS(R),5S(R),9bR(S)-3-ethyl-5-phenyl-1,2,3a,4,5,9b-hexahydro-3H-benz[e]indole is prepared.

6. A process as claimed in claim 1, wherein (±)-3aS(R),5S(R),9bR(S)-3-n-propyl-5-phenyl-1,2,3a,4,5,9b-hexahydro-3H-benz[e]indole is prepared.

7. A process as claimed in claim 1, wherein (±)-3aS(R),5S(R),9bR(S)-3-methyl-5-m-chlorophenyl-1,2,3a,4,5,9b-hexahydro-3H-benz[e]indole.

8. A process as claimed in claim 1, wherein (±)-3aS(R),5S(R),9bR(S)-3-methyl-5-fluorophenyl-1,2,3a,4,5,9b-hexahydro-3H-benz[e]indole is prepared.

**Revendications**
**Revendications pour les Etats contractants suivants : BE, CH, DE, FR, GB, IT, NL**

1. 5-Phényl-1,2,3a,4,5,9b-hexahydro-3H-benzo[e]indoles de formule I

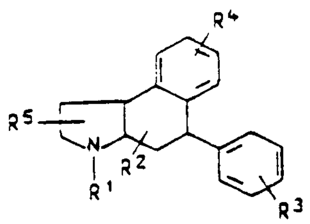

I.

dans laquelle

| | |
|---|---|
| $R^1$ | représente un atome d'hydrogène ou un reste alkyle en $C_1$-$C_6$, |
| $R^2$ | représente un atome d'hydrogène ou un reste alkyle en $C_1$-$C_3$ en position 3a ou 4, |
| $R^3$ et $R^4$ | représentent chacun un atome d'hydrogène ou d'halogène ou un reste hydroxy, alkyle en $C_1$-$C_3$, alcoxy en $C_1$-$C_3$, alkylthio en $C_1$-$C_3$ ou trifluorométhyle, acétamino, amino, et |
| $R^5$ | représente un atome d'hydrogène ou un reste alkyle en $C_1$-$C_3$, |

et leurs sels avec des acides acceptables physiologiquement

2. Composés de formule I selon la revendication 1, dans lesquels

| | |
|---|---|
| $R^1$ | représente un atome d'hydrogène ou un reste méthyle, éthyle ou n-propyle, |
| $R^2$, $R^4$ et $R^5$ | représentent chacun un atome d'hydrogène et |
| $R^3$ | représente un atome d'hydrogène, de fluor ou de chlore ou un reste trifluorométhyle, méthoxy, thiométhyle, méthyle, hydroxy, acétamino ou amino. |

3. (±)-3aS(R),5S(R),9bR(S)-3-Méthyl-5-phényl-1,2,3a,4,5,9b-hexahydro-3H-benzo[e]indole selon la revendication 1.

4. (+)-3aS,5S,9bR-3-Méthyl-5-phényl-1,2,3a,4,5,9b-hexahydro-3H-benzo[e]indole selon la revendication 1.

5. (±)-3aS(R),5S(R),9bR(S)-3-Ethyl-5-phényl-1,2,3a,4,5,9b-hexahydro-3H-benzo[e]indole selon la revendication 1.

6. (±)-3aS(R),5S(R),9bR(S)-3-n-Propyl-5-phényl-1,2,3a,4,5,9b-hexahydro-3H-benzo[e]indole selon la revendication 1.

7. (±)-3aS(R),5S(R),9bR(S)-3-Méthyl-5-m-chlorophényl-1,2,3a,4,5,9b-hexahydro-3H-benzo[e]indole selon la revendication 1.

8. (±)-3aS(R),5S(R),9bR(S)-3-Méthyl-5-m-fluorophényl-1,2,3a,4,5,9b-hexahydro-3H-benzo[e]indole selon la revendication 1.

**9.** Agent thérapeutique, contenant un composé de formule I selon la revendication 1.

**10.** Composés de formule I selon la revendication 1, destinés a être utilisés dans la lutte contre des maladies.

**Revendications pour les Etats contractants suivants : AT, ES**

**1.** Procédé de préparation des 5-phényl-1,2,3a,4,5,9b-hexahydro-3H-benzO[e]-indoles de formule I

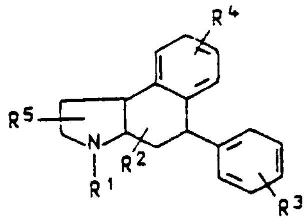

I.

dans laquelle

| | |
|---|---|
| $R^1$ | représente un atome d'hydrogène ou un reste alkyle EN $C_1$-$C_6$, |
| $R^2$ | représente un atome d'hydrogène ou un reste alkyle en $C_1$-$C_3$ en position 3a ou 4, |
| $R^3$ et $R^4$ | représentent chacun un atome d'hydrogène ou d'halogène ou un reste hydroxy, alkyle en $C_1$-$C_3$, alcoxy en $C_1$-$C_3$, alkylthio en $C_1$-$C_3$ ou trifluorométhyle, acétamino, amino, et |
| $R^5$ | représente un atome d'hydrogène ou un reste alkyle en $C_1$-$C_3$, |

et de leurs sels avec des acides acceptables physiologiquement,
caractérisé en ce que

a) on cyclise un composé de formule II

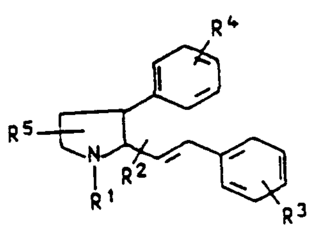

II.

dans laquelle $R^1$-$R^5$ ont les significations indiquées, en présence d'un acide fort, ou

b) on réduit un composé de formule III

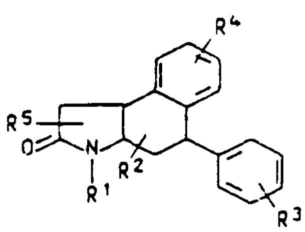

III.

dans laquelle $R^1$-$R^5$ ont les significations indiquées,

puis on transforme éventuellement les composés ainsi obtenus en leurs sels avec des acides acceptables physiologiquement.

**2.** Procédé selon la revendication 1, caractérisé en ce qu'on prépare des composés de formule I dans lesquels

| | |
|---|---|
| $R^1$ | représente un atome d'hydrogène ou un reste méthyle, éthyle ou n-propyle, |
| $R^2$, $R^4$ et $R^5$ | représentent chacun un atome d'hydrogène et |
| $R^3$ | représente un atome d'hydrogène, de fluor ou de chlore ou un reste trifluorométhy- |

le, méthoxy, thiométhyle, méthyle, hydroxy, acétamino ou amino.

3. Procédé selon la revendication 1, caractérisé en ce qu'on prépare du (±)-3aS(R),5S(R),9bR(S)-3-méthyl-5-phényl-1,2,3a,4,5,9b-hexahydro-3H-benzo[e]indole.

4. Procédé selon la revendication 1, caractérisé en ce qu'on prépare du (+)-3aS,5S,9bR-3-méthyl-5-phényl-1,2,3a,4,5,9b-hexahydro-3H-benzo[e]indole.

5. Procédé selon la revendication 1, caractérisé en ce qu on prépare du (±)-3aS(R),5S(R),9bR(S)-3-éthyl-5-phényl-1,2,3a,4,5,9b-hexahydro-3H-benzo[e]indole.

6. Procédé selon la revendication 1, caractérisé en ce qu'on prépare du (±)-3aS(R),5S(R),9bR(S)-3-n-propyl-5-phényl-1,2,3a,4,5,9b-hexahydro-3H-benzo[e]indole.

7. Procédé selon la revendication 1, caractérisé en ce qu'on prépare du (±)-3aS(R),5S(R),9bR(S)-3-méthyl-5-m-chloro-phényl-1,2,3a,4,5,9b-hexahydro-3H-benzo[e]indole.

8. Procédé selon la revendication 1, caractérisé en ce qu'on prépare du (±)-3aS(R),5S(R),9bR(S)-3-méthyl-5-fluoro-phényl-1,2,3a,4,5,9b-hexahydro-3H-benzo[e]indole.